# EUROPÄISCHE PATENTANMELDUNG

(11) **EP 3 699 924 A1**
(43) Veröffentlichungstag der Anmeldung: **26.08.2020**
(21) Anmeldenummer: 20153808.9
(22) Anmeldetag: 27.01.2020
(51) Int. Cl.: G16H 40/40

(54) **MEDIZINGERÄT UND MEDIZINGERÄTESYSTEM**

(30) Priorität: 25.02.2019 DE 102019104707
(71) Anmelder: OLYMPUS WINTER & IBE GMBH, 22045 Hamburg (DE)
(72) Erfinder: RAMIN, Daniel, 14558 Nuthetal (DE); DIETRICH, Stefan, 12169 Berlin (DE)
(74) Vertreter: Noack, Andreas

(57) **Zusammenfassung**

Es wird ein Medizingerät vorgestellt, umfassend eine medizinische Funktionseinheit (7), eine elektronische Steuereinheit (6), und eine Spannungsversorgung (5) für die medizinische Funktionseinheit (7) und/oder die elektronische Steuereinheit (6), wobei die Spannungsversorgung (5) eingerichtet ist, an ein drahtgebundenes externes Versorgungsspannungsnetzwerk (2) angeschlossen zu werden, und wobei die elektronische Steuereinheit (6) eine Datenschnittstelle (10) umfasst, um Daten mit einem externen Gerät (20) auszutauschen Das Medizingerät zeichnet sich dadurch aus, dass die Datenschnittstelle (10) eingerichtet ist, Daten über wenigstens einen Leiter des Versorgungsspannungsnetzwerks (2) zu senden und/oder zu empfangen.

## Beschreibung

Die Erfindung betrifft ein Medizingerät, umfassend eine medizinische Funktionseinheit, eine elektronische Steuereinheit, und eine Spannungsversorgung für die medizinische Funktionseinheit und/oder die elektronische Steuereinheit, wobei die Spannungsversorgung eingerichtet ist, an ein drahtgebundenes externes Versorgungsspannungsnetzwerk angeschlossen zu werden, und wobei die elektronische Steuereinheit eine Datenschnittstelle umfasst, um Daten mit einem externen Gerät auszutauschen.

Weiterhin betrifft die Erfindung ein Medizingerätesystem mit einem solchen Medizingerät.

Gattungsgemäße Medizingeräte sind aus der modernen Medizin nicht mehr wegzudenken. Sie weisen neben einer medizinischen Funktionseinheit, welche die eigentliche medizinische Funktionalität bereitstellt, eine elektronische Steuereinheit auf, welche verschiedenste Aufgaben übernimmt. Neben der Steuerung der eigentlichen medizinischen Funktionseinheit übernimmt die elektronische Steuerung unter anderem die Kommunikation dem Benutzer über eine Benutzerschnittstelle, und gegebenenfalls die Kommunikation mit anderen Medizingeräten und mit Patientendaten-Verwaltungssystemen.

Um die Funktionsbereitschaft solcher Medizingeräte aufrecht zu erhalten muss die elektronische Steuereinheit regelmäßig gewartet werden. Dazu wird die elektronische Steuereinheit über eine Datenschnittstelle mit einem externen Gerät, beispielsweise einem Wartungsgerät, verbunden, um mit diesem Daten auszutauschen. Bei den Daten kann es sich um Betriebs- oder Diagnosedaten des Medizingeräts handeln, welche zur Erkennung oder Untersuchung von Fehlfunktionen durch das Wartungsgerät ausgewertet werden können. Weiterhin kann es sich bei den Daten um geänderte Konfigurationsdaten handeln, mit welchen die Funktionalität des Medizingeräts angepasst werden kann. Ebenso kann es sich bei den Daten um neue oder geänderte Betriebssoftware handeln, welche durch die elektronische Steuereinheit ausgeführt werden soll. Die Datenschnittstelle kann beispielsweise als USB- oder Ethernet-Schnittstelle ausgeführt sein.

Eine offene Datenschnittstelle ist aus verschiedenen Gründen nicht unproblematisch. Zum einen stellt eine solche Datenschnittstelle ein Sicherheitsrisiko dar, da über sie theoretisch ein unbefugter Zugriff auf die elektronische Steuereinheit des Medizingeräts ermöglicht wird. Ein solcher Zugriff kann in dem Auslesen vertraulicher Patientendaten oder Konfigurationsdaten bestehen. Ebenso kann über einen unbefugten Zugriff die Konfiguration oder die Betriebssoftware der elektronischen Steuereinheit manipuliert werden, was zu einem Ausfall oder einer Fehlfunktion führen kann.

Ein weiteres Problem besteht darin, dass eine offene Schnittstelle schwer zu reinigen ist und somit ein Kontaminationsrisiko darstellt.

Den geschilderten Problemstellungen wurde bislang unter anderem dadurch begegnet, dass die Datenschnittstelle eines Medizingeräts im Inneren eines geschlossenen Gehäuses des Medizingeräts vorgesehen wurde. Um auf die Schnittstelle zuzugreifen musste dabei das Medizingerät vorzugsweise aus einem Operationssaal entfernt werden, um dass das Gehäuse zu öffnen und das Wartungsgerät anzuschließen. Während durch die beschriebene Ausführung eines Medizingeräts die geschilderten Problematiken weitgehend beseitigt sind, ist die Handhabung eines entsprechenden Medizingeräts während der Wartung schwierig. Gleichzeitig stellt der Betrieb eines Medizingeräts mit geöffnetem Gehäuse ein Unfallrisiko für den Bediener dar, da spannungführende Teile, z.T. auch Hochspannungsführende Teile, offen liegen können. Zusätzlich erfordert die Wiederinbetriebnahme eines Medizingeräts nach zwischenzeitlicher Öffnung des Gehäuses eine umfangreiche Funktionsprüfung.

Es besteht daher eine Aufgabe der Erfindung darin, ein Medizingerät und ein Medizingerätesystem bereitzustellen, die hinsichtlich der beschriebenen Problematik verbessert sind.

Diese Aufgabe wird gemäß eines Aspekts der Erfindung gelöst durch ein Medizingerät, umfassend eine medizinische Funktionseinheit, eine elektronische Steuereinheit, und eine Spannungsversorgung für die medizinische Funktionseinheit und/oder die elektronische Steuereinheit, wobei die Spannungsversorgung eingerichtet ist, an ein drahtgebundenes externes Versorgungsspannungsnetzwerk angeschlossen zu werden, und wobei die elektronische Steuereinheit eine Datenschnittstelle umfasst, um Daten mit einem externen Gerät auszutauschen, welches dadurch weitergebildet ist, dass die Datenschnittstelle eingerichtet ist, Daten über wenigstens einen Leiter des Versorgungsspannungsnetzwerks zu senden und/oder zu empfangen.

Durch die entsprechende Einrichtung der Datenschnittstelle sind neben einer Anschlussvorrichtung zum Anschluss an das Versorgungsspannungsnetzwerk, welche an dem Medizingerät ohnehin vorhanden sein muss, keine weiteren Anschlussvorrichtungen an dem Medizingerät erforderlich, wodurch sowohl die Datensicherheit als auch die Reinigbarkeit des Medizingeräts verbessert ist. Diese Aussage bezieht sich ausschließlich auf Datenschnittstellen. Dabei kann das Medizingerät weitere Schnittstellen aufweisen, an welche Applikatoren oder andere Instrumente angeschlossen werden können.

In einer möglichen Umsetzung eines Medizingeräts nach der Erfindung kann die Datenschnittstelle eine Modulations- und/oder Demodulationseinrichtung umfassen, mittels derer ein Datensignal auf ein Versorgungssignal des Versorgungsspannungsnetzwerks aufmoduliert werden kann, und/oder ein auf dem Versorgungssignal des Versorgungsspannungsnetzwerks aufmoduliertes Datensignal von diesem demoduliert werden kann. Auf diese Weise kann das Datensignal unabhängig von einer ggf. im Versorgungsspannungsnetzwerk vorherrschenden Netzfrequenz übertragen werden.

Die Modulationsart und die Modulationsfrequenz können je nach gewünschter Bandbreite und Reichweite der Datenübertragung gewählt werden. Dabei kann mit einer hohen Modulationsfrequenz eine höhere Datenrate übertragen werden, wobei gleichzeitig eine höhere Bedämpfung des Signals in den nicht für hohe Frequenzen ausgelegten Leitungen des Versorgungsspannungsnetzwerks erfolgt, so dass nur geringe Distanzen überbrückt werden können. Sollen hingegen größere Distanzen überbrückt werden, kann eine kleinere Trägerfrequenz gewählt werden, wobei auch nur eine geringere Datenrate zur Verfügung steht.

In einer vorteilhaften Weiterbildung eines Medizingeräts nach der Erfindung kann die Modulations- und/oder Demodulationseinrichtung wenigstens ein Frequenzfilter umfassen. Das Frequenzfilter kann die Netzfrequenz, welche beispielsweise bei etwa 50Hz liegen kann, von der Modulationsfrequenz trennen, die beispielsweise bei einigen 100 kHz liegen kann.

Die Datenschnittstelle eines Medizingeräts kann in einer Ausführungsform der Erfindung eine Speichereinrichtung umfassen, um über die Datenschnittstelle empfangende oder zu sendende Daten zwischenzuspeichern. Im Vergleich zu dezidierten Datennetzwerken muss bei der Übertragung von Daten über ein Versorgungsspannungsnetzwerk mit deutlich mehr Störsignalen durch weitere angeschlossene Verbraucher gerechnet werden.

Um eine reibungslosen Austausch der Daten mit der elektronischen Steuereinheit zu gewährleisten werden hier vom Medizingerät zu sendende Daten zunächst in einem schnellen und wenig störanfälligen Datentransfer in die Speichereinrichtung übertragen, und dann von der Datenschnittstelle je nach verfügbarer Bandbreite über das Versorgungsspannungsnetzwerk ausgegeben. Umgekehrt werden durch das Medizingerät zu empfangende Daten erst in der Speichereinrichtung abgelegt. Sobald die zu empfangenden Daten vollständig in der Speichereinrichtung abgelegt sind, werden diese komplett an die elektronische Steuereinheit übertragen.

Um die Gefahr von Datenfehlern durch Störsignale zu reduzieren empfiehlt es sich, zur Datenübertragung ein geeignetes fehlererkennendes oder fehlerkorrigierendes Datenprotokoll zu verwenden.

Die über die Datenschnittstelle zu sendenden Daten Betriebsdaten und/oder Diagnosedaten umfassen. Die über die Datenschnittstelle zu empfangenden Daten Konfigurationsdaten, und/oder Betriebssoftware umfassen.

Bei einem Medizingeräts gemäß einer besonders bevorzugten Ausführung der Erfindung kann die Datenschnittstelle eingerichtet sein, Daten vor der Übertragung zu verschlüsseln und/oder Daten nach dem Empfang zu entschlüsseln. Eine Verschlüsselung der Daten ist sinnvoll, da die Datenübertragung durch das Versorgungsspannungsnetzwerk leicht ausgespäht werden kann, beispielsweise über eine Versorgungssteckdose in öffentlich zugänglichen Bereich eines Krankenhauses, in welchem das Medizingerät eingesetzt ist. Insbesondere bei der Übertragung von vertraulichen Daten, wie z.B. persönlichkeitsrelevanten Patientendaten, ist eine Verschlüsselung daher wünschenswert. Für die Verschlüsselung können prinzipiell beliebige bekannte symmetrische oder asymmetrische Verschlüsselungsprotokolle verwendet werden.

In einer möglichen Ausführung der Erfindung kann die Datenschnittstelle eingerichtet sein, in Reaktion auf ein Anforderungssignal Daten zu übertragen.

Die medizinische Funktionseinheit eines Medizingeräts gemäß der Erfindung kann zur Ausführung unterschiedlicher medizinischer Funktionen eingerichtet sein. Dabei kann es sich bei dem Medizingerät um eine Spülpumpe, einen Insufflator, eine Videokontrolleinheit, oder einen Ultraschallgenerator handeln. In einer bevorzugten Ausführung der Erfindung kann das Medizingerät ein elektrochirurgischer Generator sein.

Die Aufgabe wird ferner gemäß eines weitere Aspekts der Erfindung gelöst durch ein Medizingerätesystem, umfassend ein Medizingerät nach den obigen Ausführungen, und ein Wartungsgerät, das eingerichtet ist, an ein drahtgebundenes Versorgungsspannungsnetzwerk angeschlossen zu werden, wobei das Wartungsgerät eine Datenschnittstelle umfasst, die eingerichtet ist, über wenigstens einen Leiter des Versorgungsspannungsnetzwerks Daten mit dem Medizingerät auszutauschen.

Gemäß eines zusätzlichen Aspekts der Erfindung wird die Aufgabe weiterhin gelöst durch ein Medizingerätesystem, umfassend: ein erstes Medizingerät, und wenigstens ein zweites Medizingerät, wobei das erste Medizingerät und das wenigstens eine zweite Medizingerät jeweils nach einem der Erfindung ausgeführt und eingerichtet sind, Daten untereinander auszutauschen.

Bezüglich der hierdurch erreichbaren Wirkungen und Vorteile wird auf das oben bereits gesagte verwiesen.

Der Begriff "medizinische Funktionseinheit" ist im Sinne der Erfindung so auszulegen, dass es sich dabei um eine logische und/oder physikalische Einheit handelt, welche die Hauptfunktion des Medizingeräts bereitstellt. Dabei muss diese Hauptfunktion nicht zwangsläufig eine unmittelbar medizinische Funktion sein, so kann z.B. bei einem als Medizingerät zu betrachtenden Videomonitor die medizinische Funktionseinheit Baugruppen zum Empfangen, Dekodieren und Darstellen eines Videosignals umfassen, auch wenn diese Baugruppen ebenso in nichtmedizinischen Videomonitoren vorhanden sind.

Die medizinische Funktionseinheit und die elektronische Steuereinheit müssen nicht zwangsläufig getrennte Komponenten eines Medizingeräts nach der Erfindung sein, sondern können auch teilweise oder vollständig identische Komponenten umfassen.

Die Erfindung wird nachfolgend anhand einiger Figuren näher erläutert, wobei die Figuren und Ausführungsbeispiele lediglich zum besseren Verständnis der Erfindung beitragen sollen, ohne diese einzuschränken.

Es zeigen:
- Fig. 1:: ein Medizingerätesystem.
- Fig. 2:: ein weiteres Medizingerätesystem.

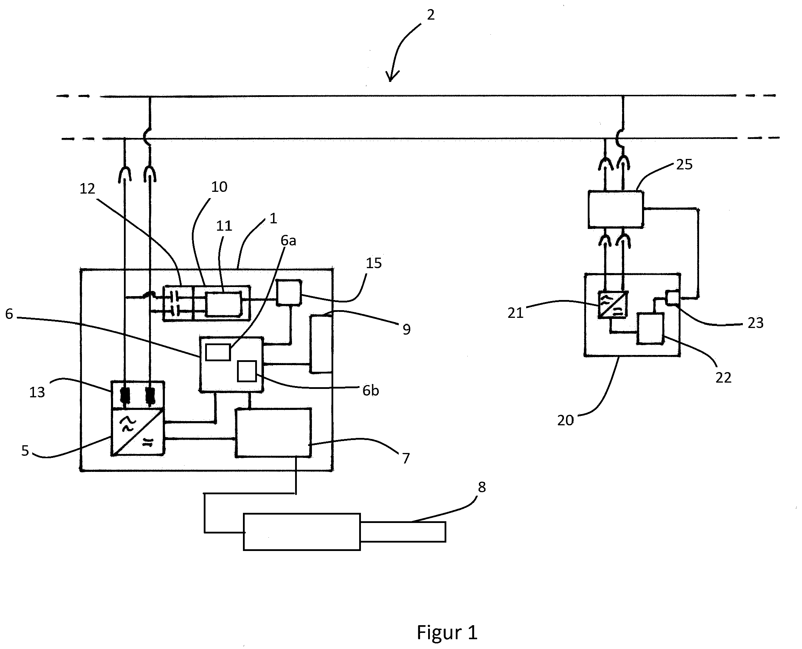

Die Figur 1 zeigt ein Medizingerätesystem mit einem Medizingerät 1, welches an ein Versorgungsspannungsnetzwerk 2 angeschlossen ist. Das Versorgungsspannungsnetzwerk ist als zweiadriges Netzwerk (Phase + Nullleiter) dargestellt, kann aber ebenso mit mehreren Adern (3 Phasen + Nullleiter), oder fünf Adern (3 Phasen + Nullleiter + Schutzleiter).

Das Medizingerät 1 weist weiterhin ein Spannungsversorgung 5 in Form eines Netzteils, eine elektronische Steuereinheit 6, sowie eine medizinische Funktionseinheit 7 auf. An die medizinische Funktionseinheit 7 ist im dargestellten Beispiel ein Instrument 8 angeschlossen.

Die elektronische Steuereinheit 6 steuert die Funktion der medizinischen Funktionseinheit 7, und stellt gleichzeitig eine Benutzerschnittstelle 9 zur Verfügung, über welche ein Benutzer das Medizingerät 1 bedienen kann. Die Benutzerschnittstelle 9 kann einen Touchscreen beinhalten, und oder separate Bedienelemente wie Drehsteller oder Schalter aufweisen. Die Benutzerschnittstelle 9 kann auch externe Bedienelemente umfassen, wie beispielsweise Fußschalter (nicht dargestellt).

Je nach Art des Medizingeräts 1 stellt die medizinische Funktionseinheit 7 eine oder mehrere medizinische Funktionalitäten bereit. Das Medizingerät 1 kann beispielsweise ein elektrochirurgischer Generator, ein Insufflator, ein Endoskop-Steuergerät, ein Ultraschallgenerator, oder eine Spülpumpe sein oder umfassen.

Die Steuereinheit 6 umfasst einen Mikroprozessor 6a und eine Speichereinheit 6b, auf welcher Programmbefehle für den Mikroprozessor 6a sowie Konfigurationsdaten des Medizingeräts 1 abgelegt sind. Zudem ermittelt die Steuerung 6 im Betrieb des Medizingeräts 1 Betriebs- und Diagnosedaten, welche ebenfalls Um die Betriebsbereitschaft des Medizingeräts 1 zu erhalten muss die Steuereinheit 6 gewartet werden, dabei werden Betriebs- und/oder Diagnosedaten ausgelesen und gegebenenfalls aktualisierte Programmbefehle und/oder Konfigurationsdaten in der Speichereinheit 6b abgelegt.

Für die im Rahmen der Wartung erforderliche Datenkommunikation zu ermöglichen, weist das Medizingerät 1 eine Datenschnittstelle 10 auf. Die Datenschnittstelle 10 ist eingerichtet, Daten über wenigstens einen Leiter des Spannungsversorgungsnetzwerks 2 zu senden und/oder zu empfangen. Dazu weist die Datenschnittstelle eine Modulations- und/oder Demodulationseinrichtung (Modem) 11 auf, welche zum Senden von Daten ein Datensignal auf eine hochfrequente Trägerfrequenz aufmoduliert und das resultierende Signal auf in die Leiter des Versorgungsspannungsnetzwerks 2 einspeist. Zum Empfangen von Daten greift das Modem 11 die Trägerfrequenz mit dem Datensignal von den Leitern des Versorgungsspannungsnetzwerks 2 ab, und trennt das Datensignal anschließend von der Trägerfrequenz.

Um die Trägerfrequenz und eine Grundfrequenz des Versorgungsspannungsnetzwerks 2 zu trennen sind Frequenzfilter 12, 13 vorgesehen. Dabei ist das Filter 12 Bestandteil des Modems 11, während das Filter 13 ein Schutzfilter der Spannungsversorgung 5 ist.

Für die hier beschriebene Datenübertragung über das Versorgungsspannungsnetzwerk 2 werden zwei Leiter des Versorgungsspannungsnetzwerks 2 verwendet. Es sich aber auch Datenübertragungsverfahren denkbar, die nur einen oder mehr als zwei Leiter verwenden. Ebenso kann die Anzahl der für die Datenübertragung verwendeten Leiter des Versorgungsspannungsnetzwerks 2 von der Gesamtzahl der Leiter des Versorgungsspannungsnetzwerks 2 abweichen.

Die Datenschnittstelle 10 umfasst weiterhin eine Speichereinrichtung 15 zum Zwischenspeichern von empfangenen und/oder zu sendenden Daten.

Die Datenschnittstelle 10 kann in dem Medizingerät 1 als separate Baugruppe vorgesehen sein. Alternativ kann die Datenschnittstelle ganz oder teilweise gemeinsam mit anderen Komponenten auf einer Platine angeordnet sein, beispielsweise zusammen mit der Steuereinheit 6. Prinzipiell ist es auch möglich, die Datenschnittstelle 10 außerhalb eines Gehäuses des Medizingeräts 1 anzuordnen.

Das Medizingerätesystem umfasst weiterhin ein Wartungsgerät 20, welches ebenfalls an das Versorgungsspannungsnetzwerk 2 angeschlossen ist. Das Wartungsgerät 20 umfasst ein Netzteil 21 sowie eine Wartungseinheit 22, die beispielsweise als PC ausgeführt ist. Das Wartungsgerät weist eine standardisierte Datenschnittstelle 23 auf, bei welcher es sich um eine USB- oder Ethernet-Schnittstelle handeln kann. Um über das Versorgungsspannungsnetzwerk 2 Daten mit dem Medizingerät 1 auszutauschen ist die Datenschnittstelle 23 des Wartungsgeräts 20 über einen Adapter 25 mit dem Versorgungsspannungsnetzwerk 2 verbunden, welcher ähnlich aufgebaut ist wie die Datenschnittstelle 10.

Bei der Datenübertragung über die Leitungen des Versorgungsspannungsnetzwerks 2 können Störungssignale auftreten, die beispielsweise aus Schaltvorgängen oder Einstreuungen anderer an das Versorgungsspannungsnetzwerk angeschlossenen Verbrauchern resultieren. Gerade im Fall von elektrochirurgischen Instrumenten können diese Störungssignale eine Datenübertragung erheblich beeinträchtigen. Es ist daher vorteilhaft, bei der Datenübertragung ein fehlertolerantes Übertragungsprotokoll zu wählen.

Durch die möglichen Störeinflüsse auf die Datenübertragung kann die Übertragungsgeschwindigkeit gering sein. Um in diesem Fall die Steuereinheit 6 nicht zu lange zu blockieren ist die Datenschnittstelle 10 mit einem einer eigenen Speichereinrichtung 15 ausgestattet. In der Speichereinrichtung 15 werden empfangende oder zu sendende Daten zwischengespeichert. Sollen Daten von der Steuereinheit 6 übertragen werden, so werden diese Daten zunächst komplett in einem schnellen internen Transfer in die Speichereinrichtung 15 übertragen. Danach werden die Daten je nach verfügbarer Übertragungsrate an das Wartungsgerät 20 gesendet. Ebenso werden durch die Datenschnittstelle 10 empfangene Daten in der Speichereinrichtung 15 abgelegt, und erst nach abgeschlossener Übertragung aller Daten komplett an die Steuereinheit 6 übertragen.

Auf diese Weise wird die Steuereinheit 6 durch die Datenübertragung möglichst wenig ausgelastet, so dass sie weiterhin die medizinische Funktionseinheit 7 kontrollieren kann. Trotzdem sollte die Übertragung größerer Datenmengen vorzugsweise erfolgen, wenn das Medizingerät 1 nicht in Betrieb ist.

Üblicherweise wird die Kommunikation zwischen dem Medizingerät 1 und dem Wartungsgerät 20 durch das Wartungsgerät 20 gesteuert werden. Das heißt, dass die Datenschnittstelle 10 des Medizingeräts 1 inaktiv ist, bis das Wartungsgerät 20 über das Versorgungsnetzwerk 2 ein Aktivierungssignal sendet. Das Aktivierungssignal kann beispielsweise eine Identifikationsnummer der Datenschnittstelle 10 beinhalten, so dass nur die richtige Datenschnittstelle aktiviert wird, falls mehrere Medizingeräte an dem Spannungsversorgungsnetzwerk 2 angeschlossen sind. Das Aktivierungssignal kann weiterhin eine Information darüber beinhalten, ob die Datenschnittstelle 10 Daten senden oder empfangen soll, und um welche Daten es sich dabei handelt.

Als zu empfangende Daten kommen insbesondere aktualisierte Betriebssoftware (updates, bugfixes, neue Features, etc.) oder Konfigurationsdaten (Parameter für neue Betriebsmodi oder Instrumente, etc.) in Betracht. Als durch die Datenschnittstelle 10 zu sendende Daten kommen insbesondere Betriebsdaten (Betriebsstundenzähler, Prozedurprotokolle, etc.) oder Diagnosedaten (Fehlermeldungen, interne Messdaten, etc.) in Betracht. Da diese Daten zum Teil vertraulich sind, wird gewöhnlich ein verschlüsseltes Datenübertragungsprotokoll verwendet werden, wobei gleichermaßen symmetrische wie asymmetrische Verschlüsselungen möglich sind.

Die Verwendung des Spannungsversorgungsnetzwerks 2 als Medium für die Datenübertragung mach das Medizingerätesystem besonders flexibel. So können das Medizingerät 1 und das Wartungsgerät 20 gemeinsam in einem separaten störungsarmen System angeordnet sein, beispielsweise in einem Fertigungs- oder Wartungscenter des Herstellers der Geräte. In einem solchen störungsarmen System mit kurzen Übertragungswegen lassen sich hohe Datenraten erzielen, um z.B. eine Erstinstallation mit umfangreicher Betriebssoftware und/oder Konfigurationsdaten vorzunehmen, oder um Betriebsdaten von Inbetriebnahmetests auszulesen.

Im Feld wird das Medizingerät 1 hingegen oftmals in einer OP-Installation eingebunden sein. Hier kann das Wartungsgerät dann an einer frei zugänglichen Anschlussdose des Versorgungsspannungsnetzwerks 2 angeschlossen werden, die entweder im gleichen Raum oder aber in einem anderen Raum liegen kann. Anders als im vorbeschriebenen Einsatzfall wäre hier mit mehr Störungen zu rechnen, da ggf. in benachbarten OP-Sälen andere Medizingeräte in Betrieb sind, die an das gleicher Spannungsversorgungsnetzwerk angeschlossen sind. Da aber die Im Feld zu übertragenden Datenmengen in der Regel geringer sind als die bei der Erstinstallation anfallenden Datenmenden, kann auch in diesem Umfeld eine hinreichende Übertragungsgeschwindigkeit erreicht werden.

Für die technische Umsetzung der Erfindung kann auf prinzipiell bekannte Technologien aus dem Bereich der Trägerfrequenzanlagen zurückgegriffen werden. Auf eine detaillierte Beschreibung wird daher an dieser Stelle aus Gründen der Übersichtlichkeit verzichtet.

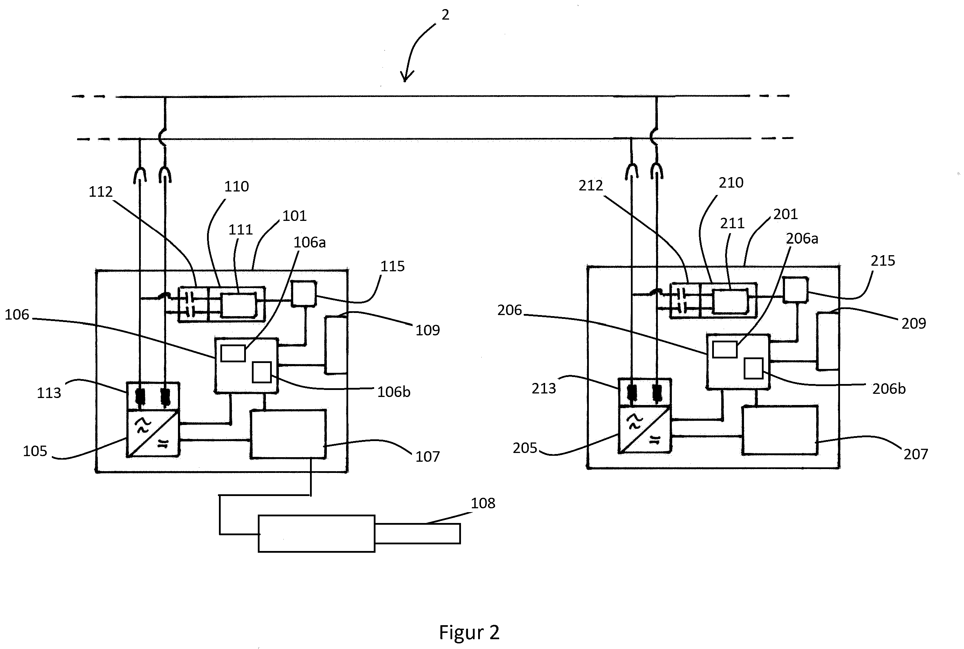

In der Figur 2 ist ein weiteres Medizingerätesystem dargestellt, welches zwei Medizingeräte 101, 201 aufweist. Der prinzipielle Aufbau der Medizingeräte 101, 201 entspricht dabei dem des Medizingeräts 1 aus Figur 1. Sich entsprechende Elemente sind daher mit einem um 100 oder 200 erhöhten Bezugszeichen versehen und werden nicht erneut erläutert.

Die Medizingeräte 101, 201 sind eingerichtet, über das Versorgungsspannungsnetzwerk 2 Daten auszutauschen. Der Datenaustausch funktioniert dabei prinzipiell genauso wie der Datenaustausch zwischen dem Medizingerät 1 und dem Diagnosegerät 20 in Figur 1.

Bei dem Medizingerät 101 kann es sich wiederum um einen elektrochirurgischen Generator handeln, das Medizingerät 201 ist vorzugsweise für eine andere medizinische Funktionalität vorgesehen. Beispielsweise kann das Medizingerät 201 ein Hilfsgerät sein, welches eine den elektrochirurgischen Generator 101 unterstützende Funktion ausführt, z.B. eine Rauchgasabsaugung oder eine Kühlmittelpumpe. Die Datenübertragung zwischen den Medizingeräten 101, 201 kann dann dazu dienen, die Funktion des Hilfsgeräts 201 mit der Funktion des elektrochirurgischen Generators 101 zu synchronisieren, also z.B. eine Rauchgasabsaugung oder eine Kühlmittelzufuhr dann zu aktivieren, wenn der elektrochirurgische Generator 101 über das Instrument 108 Energie abgibt.

Das Medizingerät 201 kann beispielsweise auch ein Videomonitor sein, auf welchem während einer Prozedur das Bild einer Raumkamera, einer Boom-Arm-Kamera, oder einer endoskopischen Kamera dargestellt wird. Das Medizingerät 101 kann dann Betriebsdaten wie Betriebsmodus, Aktivierungsstatus, oder eingestellte Parameter über das Versorgungsspannungsnetzwerk 2 an den Videomonitor 201 senden, welcher dann die empfangenen Betriebsdaten als Overlay in das Videobild einblenden kann.

Das Medizingerät 201 kann auch ein Medizingeräte-Controller sein, welcher eine zentrale Bedienplattform bereitstellt, um mehrere andere Medizingeräte zu steuern. In diesem Fall können über das Versorgungsspannungsnetzwerk 2 Betriebsdaten von dem Medizingerät 101 an den Medizingerätecontroller gesendet werden, parallel können Steuerbefehle von dem Medizingerätecontroller an das Medizingerät 101 gesendet werden.

Bei dem in Figur 2 dargestellten Medizingerätesystem tritt die Besonderheit auf, dass eine Datenübertragung über das Versorgungsspannungsnetzwerk 2 auch dann wünschenswert ist, wenn die Medizingeräte 101, 201 aktiviert sind. Insbesondere bei Medizingeräten wie elektrochirurgischen Generatoren ist mit dem Auftreten nennenswerter Störsignale zu rechnen, welche über die Spannungsversorgung 105 in das Versorgungsspannungsnetzwerk 2 eingekoppelt werden. Dies können zum einen hochfrequente Störspannungen aus dem HF-Ausgangssignal des elektrochirurgischen Generators sein, zum anderen können aus einer stark schwankenden Stromaufnahme der Spannungsversorgung 105, z.B. bei gepulsten Betriebsmodi des elektrochirurgischen Generators, Verzerrungen des Spannungsverlaufs des Versorgungsspannungsnetzwerks 2 erfolgen.

Um trotz der beschriebenen Störsignale eine stabile Datenübertragung zu ermöglichen stehen verschiedene Methoden zur Verfügung. So kann eine störende Auswirkung hochfrequenter Störsignale dadurch vermieden werden, dass eine für die Datenübertragung gewählte Trägerfrequenz deutlich außerhalb, vorzugsweise unterhalb, einer Grundfrequenz der Störsignale liegt. So kann beispielsweise bei Störsignalen im Frequenzbereich von einigen hundert kHz eine Übertragungsfrequenz im Bereich von einigen 10 kHz, bspw. 30kHz, verwendet werden. Eine solche Übertragungsfrequenz reicht aus, um lediglich Betriebs- und/oder Statusdaten zwischen den Medizingeräten 101, 201 zu übertragen. Weiterhin kann insbesondere bei gepulsten Betriebsmodi eines elektrochirurgischen Generators die Datenübertragung auf die Pulspausen beschränkt werden.

Phasenverzerrungen in dem Versorgungsspannungsnetzwerk 2 können kompensiert werden, indem die Datenübertragung auf wenig verzerrte Phasenabschnitte einer Netzgrundfrequenz in dem Versorgungsspannungsnetzwerk 2 beschränkt wird. Ebenso kann eine Beeinflussung des Phasenverlaufs des Versorgungsspannungsnetzwerks 2 durch geeignete Maßnehmen zur Leistungsfaktorkorrektur reduziert werden, z.B. durch den Einsatz von Leistungsfaktorkorrekturfiltern.

## Patentansprüche

1. Medizingerät, umfassend:
- eine medizinische Funktionseinheit (7),
- eine elektronische Steuereinheit (6), und
- eine Spannungsversorgung (5) für die medizinische Funktionseinheit (7) und/oder die elektronische Steuereinheit (6),
wobei die Spannungsversorgung (5) eingerichtet ist, an ein drahtgebundenes externes Versorgungsspannungsnetzwerk (2) angeschlossen zu werden, und
wobei die elektronische Steuereinheit (6) eine Datenschnittstelle (10) umfasst, um Daten mit einem externen Gerät (20) auszutauschen,
**dadurch gekennzeichnet, dass** die Datenschnittstelle (10) eingerichtet ist, Daten über wenigstens einen Leiter des Versorgungsspannungsnetzwerks (2) zu senden und/oder zu empfangen.

2. Medizingerät nach Anspruch 1, **dadurch gekennzeichnet, dass** die Datenschnittstelle (10) eine Modulations- und/oder Demodulationseinrichtung (11) umfasst, mittels derer ein Datensignal auf ein Versorgungssignal des Versorgungsspannungsnetzwerks (2) aufmoduliert werden kann, und/oder ein auf dem Versorgungssignal des Versorgungsspannungsnetzwerks (2) aufmoduliertes Datensignal von diesem demoduliert werden kann.

3. Medizingerät nach Anspruch 2, **dadurch gekennzeichnet, dass** die Modulations- und/oder Demodulationseinrichtung (11) wenigstens ein Frequenzfilter (12) umfasst.

4. Medizingerät nach einem der vorangehenden Ansprüche, **dadurch gekennzeichnet, dass** die Datenschnittstelle (10) eine Speichereinrichtung (15) umfasst, um über die Datenschnittstelle (10) empfangende oder zu sendende Daten zwischenzuspeichern.

5. Medizingerät nach einem der vorangehenden Ansprüche, **dadurch gekennzeichnet, dass** die über die Datenschnittstelle (10) zu sendenden Daten Betriebsdaten und/oder Diagnosedaten umfassen.

6. Medizingerät nach einem der vorangehenden Ansprüche, **dadurch gekennzeichnet, dass** die über die Datenschnittstelle (10) zu empfangenden Daten Konfigurationsdaten, und/oder Betriebssoftware umfassen.

7. Medizingerät nach einem der vorangehenden Ansprüche, **dadurch gekennzeichnet, dass** die Datenschnittstelle (10) eingerichtet ist, Daten vor der Übertragung zu verschlüsseln und/oder Daten nach dem Empfang zu entschlüsseln.

8. Medizingerät nach einem der vorangehenden Ansprüche, **dadurch gekennzeichnet, dass** die Datenschnittstelle (10) eingerichtet ist, in Reaktion auf ein Anforderungssignal Daten zu übertragen.

9. Medizingerät nach einem der vorangehenden Ansprüche, **dadurch gekennzeichnet, dass** das Medizingerät ein elektrochirurgischer Generator ist.

10. Medizingerätesystem, umfassend:
- ein Medizingerät (1) nach einem der vorangehenden Ansprüche, und
- ein Wartungsgerät (20), das eingerichtet ist, an ein drahtgebundenes Versorgungsspannungsnetzwerk (2) angeschlossen zu werden,
wobei das Wartungsgerät (20) eine Datenschnittstelle (23, 25) umfasst, die eingerichtet ist, über wenigstens einen Leiter des Versorgungsspannungsnetzwerks (2) Daten mit dem Medizingerät (1) auszutauschen.

11. Medizingerätesystem, umfassend:
- ein erstes Medizingerät (101), und
- wenigstens ein zweites Medizingerät (201),
wobei das erste Medizingerät (201) und das wenigstens eine zweite Medizingerät (201) jeweils nach einem der Ansprüche 1 bis 9 ausgeführt und eingerichtet sind, Daten untereinander auszutauschen.
